Europäisches Patentamt

European Patent Office

Office européen des brevets

(19)

(11) Publication number: **0 100 914**

**B1**

(12) **EUROPEAN PATENT SPECIFICATION**

(45) Date of publication of patent specification: **29.10.86**

(21) Application number: **83106962.0**

(22) Date of filing: **15.07.83**

(51) Int. Cl.⁴: **G 01 N 33/543,**
**G 01 N 33/574**

(54) **Method of determining immune complexes specific to tumor associated antigen.**

(30) Priority: **16.07.82 JP 124945/82**

(43) Date of publication of application:
**22.02.84 Bulletin 84/08**

(45) Publication of the grant of the patent:
**29.10.86 Bulletin 86/44**

(84) Designated Contracting States:
**DE FR IT NL**

(56) References cited:
**EP-A-0 008 432**
**EP-A-0 040 058**
**EP-A-0 057 236**
**WO-A-81/02469**
**WO-A-82/01593**
**GB-A-2 001 171**

(73) Proprietor: **OTSUKA PHARMACEUTICAL CO., LTD.**
**9, Kandatsukasacho 2-chome**
**Chiyoda-ku Tokyo 101 (JP)**

(72) Inventor: **Ishii, Masaru**
**7/6, Higashi-Oomiya 3-chome**
**Oomiya-shi Saitama-Ken (JP)**
Inventor: **Akazawa, Shugo**
**919/42, Koshikiya**
**Ageo-shi Saitama-ken (JP)**

(74) Representative: **Vossius & Partner**
**Siebertstrasse 4 P.O. Box 86 07 67**
**D-8000 München 86 (DE)**

Courier Press, Leamington Spa, England.

## Description

This invention relates to a novel method of determining immune complexes specific to tumor associated antigen.

It is known that when an antibody is produced against an antigen in the living body, the antibody combines with the antigen to form an immune complex (hereinafter referred to as "IC"). Such ICs are formed in autoimmune diseases, infectious diseases and malignant tumors. The presence of ICs causes so-called IC diseases, and detection or determination of ICs, especially soluble ICs dissolved in bloods of patients with various diseases, permits diagnosis of diseases, analysis of causes of diseases and further prognosis, so that various methods of detecting and determining ICs have been proposed. Already known as such methods are physicochemical methods such as ultracentrifugation and immuno-biological methods such as anti-immunoglobulin method and methods wherein complements or cells are used. However, the conventional methods of detecting soluble ICs in the blood are merely antigen-nonspecific methods except where the pathogenic antigen is specifically known. It is known that the antigens constituting ICs are various, and such antigens are generally covered with antibodies and are therefore difficult to detect specifically. With the conventional method, therefore, instead of detecting the antigen, the amount of an antibody bound to the antigen, for example, the amount of immunoglobulin, is measured, and the measured amount of the antibody is treated as the amount of IC in the specimen. Consequently, the amount of IC thus determined is nonspecific to the antigen which is one of the components of the IC. Moreover, the immunoglobulin which is inherently present in the sample coagulates non-specifically in the measuring system, with the result that the coagulating IgG is added to the amount of IC to be measured. The amount of such IgG coagulation varies greatly from specimen to specimen and also in accordance with the measuring conditions. Furthermore, no method of distinguishing the coagulation from IC is presently available.

It has been reported that the amount of an IC in cancer is highly in proportion to the progress, i.e., metastasis, of the cancer (Theofilopoulos, A. N. et al., J. Immunol., 119, 657 (1977)). However, it is totally unclear whether the IC determined by the conventional method is a complex of cancer specific antigen and antibody. Especially in the case of cancers, frequently there occurs attendant production of an antibody which is reactive with constituents of normal cells or tissues, i.e., auto-antibody which undergoes a nonspecific reaction. In the determination of IC in such a malignant disease, therefore, the antigen constituting the IC must be identified; otherwise, it is impossible to properly determine the disease and the specificity thereof.

In WO—A—8 102 469 a solid phase anti-C3 assay for detection of immune complexes is disclosed. In this assay an anti-C3 antibody is fixed to a solid matrix, serum samples and standards are permitted to interact and the amount of immunoglobulin bound to the solid matrix through the C3-anti-C3 reaction is measured by a second reaction with a radio or enzyme-labeled anti-Ig or staphylococcal protein A.

A method of diagnosis using an attached material for binding to an immune complex, by treating the bound complex with a series of different reagents or mixtures thereof and detecting the presence or absence of reaction in each case, is known from WO 8 201 593.

An immunological assay for the determination of an immune complex in a liquid using reactants bond to a carrier is disclosed in EP—A—0 008 432.

EP—A—0 057 236 describes a method for determining tumor-associated glucose side chain (TAG) in the body fluid of mammals, a method for diagnosing tumors through the determination, and a kit for diagnosis of tumors.

A carrier-bound immunoglobulin fission product and its use in immunologic analyses is known from GB—A—2 001 171. Method for detection of oncofetal antigen, for detection of cancer, for evaluation for cancer therapy and diagnostic kit suitable for such purpose, is disclosed in EP—A—0 040 058.

In view of the foregoing situation, we have conducted intensive research to establish a technique for determining antigen-specific ICs which is capable of specifically determining an IC specific to a tumor associated antigen present in the body fluid of the patient. Consequently we have found that body fluids of patients contain complement-binding ICs wherein the constituent antigens are tumor associated antigens (tumor markers) such as BFP (basic fetoprotein), AFP (α-fetoprotein), TAG (tumor associated glycolinkage, see Published Unexamined Japanese Patent Applications (Kokai) No. 29949/1982, No. 29950/1982 and No. 29951/1982) and CEA (carcinoembryonic antigen). We have further found that such an IC, as bound to an insolubilized anti-complement, can be specifically bound to a labelled substance or such an IC alone can be specifically insolubilized and labelled and that the above object can be fulfilled by determining the specific activity of the resulting insolubilized labelled reaction product or the unreacted labelled substance. The present invention has been accomplished based on these novel findings.

The present invention provides in one embodiment a method of determining an immune complex specific to a tumor associated antigen characterized in that it comprises the steps of:

(1) reacting a complement-binding, tumor associated antigen-antibody complex present in a body fluid as an immune complex with an insolubilized anti-complement to form an immune complex-insolubilized anti-complement bound product,

(2) reacting the immune complex-insolubilized anti-complement bound product with a predetermined amount of labelled substance specifically bindable to the tumor associated antigen selected from basic fetoprotein (BFP), α-fetoprotein (AFP), tumor associated glycolinkage (TAG) or carcinoembryonic antigen (CEA) or to the antibody thereagainst contained in the bound product,

(3) separating the resulting reaction product from the unreacted labelled substance, and

(4) determining the specific activity of the reaction product or the separated unreacted reactant.

In a second embodiment the present invention provides a method of determining an immune complex specific to a tumor associated antigen characterized in that it comprises the steps of:

(1) reacting a complement-binding, tumor associated antigen-antibody complex present in a body fluid as an immune complex with an insolubilized substance specifically bindable to the tumor associated antigen selected from basic fetoprotein (BFP), α-fetoprotein (AFP), tumor associated glycolinkage (TAG) or carcinoembryonic antigen (CEA) or to the antibody thereagainst contained in the complex to form an immune complex-insolubilized substance bound product,

(2) reacting the immune complex-insolubilized substance bound product with a predetermined amount of labelled anti-complement, anti-antibody or protein A,

(3) separating the resulting reaction product from the unreacted labelled anti-complement, anti-antibody or protein A, and

(4) determining the specific activity of the reaction product or the separated unreacted reactant.

The method of the invention has not been known and provides a technique for determining ICs specific to tumor associated antigens, overcoming the difficulties heretofore encountered in the determination. According to the present invention such ICs can be determined specifically by a relatively simple procedure. Thus the present method is very useful pathologically and clinically for the examination, diagnosis and prognosis of tumors.

The present invention will be described below in greater detail.

The first embodiment of the present invention can be practiced by reacting a tumor associated antigen-antibody complex having complement binding properties and present in a body fluid as an IC with an insolubilized anti-complement to form an IC-insolubilized anti-complement bound product, reacting the bound product with a predetermined amount of a labelled substance specifically bindable to the tumor associated antigen or the antibody against the tumor associated antigen constituting the IC of the bound product, separating the reaction product from the unreacted labelled substance, and determining the specific activity of the reaction product or the unreacted labelled substance. Stated more specifically, a complement-binding tumor associated antigen-antibody complex present in a body fluid as an IC is first reacted with an insolubilized anti-complement to form an IC-insolubilized anti-complement bound product. The IC present in the body fluid and to be used in this invention for determination is one wherein the antigen is a tumor associated antigen selected from the group consisting of BFP, AFP, CEA and TAG. The IC wherein the antigen is BFP or TAG is preferred. The IC wherein the antigen is BFP, TAG or AFP has been detected by the method of the invention for the first time. Conventionally it was not known that such ICs are present in body fluids. The ICs containing the above antigens are bindable to complements, that is, have ability to activate the complement system, and are present as bound to the complement, i.e., in the form of an antigen-antibody-complement complex. According to the present invention, therefore, the IC is bound to an insolubilized anti-complement utilizing the complement binding properties of the IC.

Samples useful for the determination are various body fluids such as blood, lymph, ascites, pleural effusion and spinal fluid. Such a body fluid can be collected in the usual manner from the desired patient in need of such determination. For example when blood is used as the sample, it is preferable to use serum or plasma, which may be treated in the usual manner before use. The amount of sample to be used is usually about 0.01 to about 1.0 ml, preferably about 0.1 to about 0.3 ml.

The insolubilized anti-complement can be commercially available or prepared by chemically or physically reacting an anti-complement with an insoluble support in the usual manner. The anti-complement is not particularly limited insofar as it is an antibody against a complement such as human complement component Clq or C3. Thus anti-complements commercially available or prepared by the usual method are usable.

Examples of useful insoluble supports are cellulose powder, Sephadex, Sepharose, polystyrene, filter paper, carboxymethylcellulose, ionexchange resin, dextran, plastic film, plastic tube, nylon, glass beads, silk, polyamine-methyl vinyl ether-maleic acid copolymer, amino acid copolymer and ethylene-maleic acid copolymer. The anti-complement is insolubilized by diazo process as covalent bond process, peptide process (acide amide derivative process, carboxy chloride resin process, carbodiimide resin process, maleic anhydride derivative process, isocyanate derivative process, cyanogen bromide-activated polysaccharide process, cellulose carbonate derivative process or process wherein a condensation agent is used), alkylation process, carrier binding process with use of a cross-linking agent (such as glutaraldehyde or hexamethylene isocyanate), carrier binding process resorting to the Ugi reaction; ion binding process wherein an ion exchange resin carrier is used; or physical adsorption process wherein glass beads or porous glass is used as a carrier.

The IC is bound to the insolubilized anti-complement, for example, by reacting a sample containing the IC with the insolubilized anti-complement in about 0.2 to about 0.5 ml of a buffer solution. The reaction can be carried out at about 4 to about 37°C for about 0.5 to about 48 hours. The buffer solution is not particularly limited and includes a weakly alkaline buffer solution usually having a pH of about 7.0 to about 8.0, such as 0.5M phosphate buffer, Tris-HCl buffer or boric acid buffer. The buffer solution may have incorporated therein usual additives such as bovine serum albumin (BSA), thimerosal or NaCl.

The reaction gives an IC-insolubilized anti-complement bound product, which is then thoroughly washed with physiological saline, and thereafter reacted with a predetermined amount of labelled substance specifically bindable with the tumor associated antigen or the antibody constituting the IC of the bound product. The labelled substance specifically bindable with the tumor associated antigen is prepared by reacting in the usual manner a labelling agent with the antibody against the tumor associated antigen constituting the IC to be determined, for example, anti-BFP for BFP, anti-AFP for AFP, anti-CEA for CEA or anti-TAG for TAG. In the case of TAG, it is also possible to use as labelled substances the products obtained by reacting a labelling agent with various lectins. Examples of such lectins are those specifically bindable with terminal galactose [J.B.C. *250*, 8518—8523 (1975); Biochem. Biophys. Res. Comm., *62*, 144 (1975); Z. Immunitaetsforch, *138*, 423—433 (1969); Br. J. Exp. Pathol., *27*, 228—236 (1946); Proc. Natl. Acad. Sci. USA., *75*, No. 5, 2215—2219 (1978), Biochemistry, *13*, 196—204 (1974); Carbohydrate Research, *51*, 107—118 (1976)] such as peanut lectin (hereinafter referred to as "PNA") and castor bean (Ricinus Commuris) lectin; those specifically bindable with terminal N-acetylgalactosamine such as Dolichos bean (Dolichos biflorus) lectin, braid orange lectin, Helix pomatia lectin, lima bean (Phaseolus limensis) lectin, soy bean (Glycine max) lectin, Bauhinia bean (Bauhinia purpurea) lectin; and those specifically bindable with terminal L-fucose such as Lotus tetragonolobus lectin [Brt. J. Enp. Pathi, *34*, 94 (1953)] and Ulex europeus lectin [Boyd. W. C. and Sharpleigh, E. Blood, *9*, 1195 (1954)].

The labelled substance specifically bindable with the antibody against the tumor associated antigen is prepared by reacting a labelling agent with the tumor associated antigen per se, i.e., BFP, AFP, TAG or CEA in the usual manner. Usable as such antibodies, antigens and lectins are those commercially available or those prepared by usual methods [Masaru Ishii et al, Saishin Igaku (Modern Medicine), Vol. 36, No. 5, pp. 860—866 (1981)].

Labelling agents for giving labelled substances which are specifically bindable with the tumor associated antigen or the antibody against the tumor associated antigen are not particularly limited. Examples of useful labelling agents are usual enzymatic labelling agents such as glucoamylase, glucose oxidase, peroxidase, alkaline phosphatase, β-galactosidase and active fragments of enzymes such as hemoctapeptide; and radioactive labelling agents such as [125]I, [131]I, tritium and radioactive lithium. Labelled substances are prepared as follows. For example, enzyme-labelled substances can be prepared with use of such enzymatic labelling agents, by the binding process which uses glutaraldehyde known as a usual crosslinking agent for proteins. Especially when glucose oxidase or peroxidase is used, a process is useful wherein aldehyde is introduced by the periodic acid method into the glycolinkage of the enzyme, and the linkage is thereafter bound with the amino group of the substance to be labelled [Method in Enzymology, Vol. 37, pp. 133—136 (1975)]. Also useful is a process wherein the antibody is made into F(ab')-SH, into which maleimide is incorporated, and the resulting product is bound to the labelling agent [Journal of Biochemistry, Vol. 79, pp. 233—236 (1976) and same, Vol. 62, pp. 285—292 (1976)]. [125]I or [131]I can be used for labelling, for example, by the usual Chloramine T process [Nature, *194*, p. 495 (1962), Biochem. J., *89*, p. 114 (1963)].

The labelled substances mentioned above for use in this invention are commercially available, and such commercial products are also usable for the present invention.

The reaction between the IC-insolubilized anti-complement bound product and the labelled substance can be carried out in the same manner as the reaction between the sample and the insolubilized anti-complement.

In the present invention, the resulting reaction product thus labelled with the labelled substance is separated from the unreacted labelled substance, and one of them is checked for specific activity. The separation can be effected easily by a usual method, for example, by removing the liquid from the reaction mixture by suction and washing the remaining product at least three times with physiological saline. The specific activity of the product or the substance is measured by a method suited to the labelling agent used. For example, when an enzymatic labelling agent is used, it is caused to act on the substrate of the enzyme, and the amount of the decomposed product of the substrate is measured by a usual method, for example, in terms of absorbance. When the radioactive labelling agent is used, the radioactivity is counted. When the specific activity of the unreacted labelled substance is measured, such activity of the reaction product to be measured can be calculated easily as the difference between the specific activity of the unreacted substance and the initial specific activity of the labelled substance used.

In this way, a specific IC in the sample, i.e., only the IC containing BFP, AFP, CEA or TAG as constituent antigen can be specifically determined easily by one of the methods of the invention.

The second embodiment of the invention is practiced by reacting the IC with an insolubilized substance specifically bindable to the tumor associated antigen selected from the group consisting of BFP, AFP, TAG and CEA or the antibody thereagainst constituting the IC to form an IC-insolubilized substance bound product, reacting the bound product with a predetermined amount of labelled anti-complement, anti-antibody or protein A reactive with the complement or the antibody of the bound product, separating the reaction product from the unreacted labelled anti-complement, anti-antibody or protein A, and determining the specific activity of the reaction product or the unreacted reactant, whereby the contemplated object of the invention can be fulfilled similarly. With the second embodiment, a sample containing the same specific IC as in the case of the first embodiment is reacted with an insolubilized substance which is specifically bindable to the tumor associated antigen or the antibody against the tumor associated antigen constituting the IC. The insolubilized substance specifically bindable to the tumor associated antigen constituting the IC is the above-mentioned anti-BFP, anti-AFP, anti-CEA, anti-TAG or lectin, as fixed to a usual insoluble support. The insolubilized substance specifically bindable to the antibody constituting the IC is BFP, AFP, TAG or CEA, as fixed to a usual insoluble support. The support may be one of those already exemplified. The foregoing anti-BFP, anti-AFP, anti-TAG, anti-CEA, lectin, BFP, AFP, TAG or CEA can be fixed to the insoluble support by a known method, for example, in accordance with the same technique as described for forming the insolubilized anti-complement.

The reaction between the sample and the insolubilized substance is carried out in the same manner as the reaction between the sample and the insolubilized anti-complement, whereby only the specific IC present in the sample can be selectively obtained as an insolubilized solid.

The specific IC-insolubilized substance bound product thus obtained is then reacted with a labelled anti-complement, anti-antibody or protein A. The labelled anti-complement or labelled anti-antibody to be used is prepared by labelling with a usual labelling agent an anti-complement or anti-antibody which undergoes immunoreaction with the complement or antibody of the insolubilized IC. The anti-complement and the labelling agent may be the same as those used for the first method. The anti-antibody is not particularly limited insofar as it is an antibody against the antibody. For example when a sample derived from a human body is used for determination, anti-human immunoglobulin G is useful. The labelling method can be the same as the one usually used. In place of the labelled anti-complement or labelled anti-antibody, protein A similarly labelled is usable. Commercial protein A produced, for example, by E. Y. Laboratory can be used as such.

The reaction between the IC-insolubilized substance bound product and the labelled anti-complement, labelled anti-antibody or labelled protein A can be carried out, for example, under the same conditions as the foregoing reaction between the sample and the insolubilized anti-complement.

The labelled reaction product thus obtained and the unreacted labelled substance (labelled anti-complement, labelled anti-antibody or labelled protein A) are separated from each other and checked for specific activity in the same manner as in the first method, whereby only the specific IC in the sample can be specifically detected and determined.

In this way, the specific tumor associated antigen-containing IC in the body fluid can be determined by the present invention. This assures diagnosis of cancer from the initial stage to the terminal stage, especially detection of early cancer. Because the specific tumor associated antigen which is present in the form of an IC in the body fluid is specifically determined according to the invention, the invention is useful for diagnosing, analyzing and investigating tumor diseases and autoimmune diseases which are not detectable by the conventional technique for determining such antigens in a free form.

The method according to one embodiment of the present invention can be practiced by:

(1) reacting the complement-binding, tumor associated antigen-antibody complex present in a body fuid as an immune complex either with an insolubilized anti-complement or with an insolubilized substance specifically bindable to TAG or the antibody against TAG constituting the complex to form an immune complex-insolubilized anti-complement bound product or an immune complex-insolubilized substance bound product,

(2) reacting the immune complex-insolubilized anti-complement bound product with a predetermined amount of labelled substance specifically bindable to TAG or the antibody against TAG constituting the bound product, or reacting the immune complex-insolubilized substance bound product with a predetermined amount of labelled anti-complement, anti-antibody or protein A,

(3) separating the resulting reaction product from the unreacted labelled substance or labelled anti-complement, anti-antibody or protein A, and

(4) determining the specific activity of the reaction product or the separated unreacted reactant. Stated more specifically, the insolubilized substance specifically bindable to TAG used in the step (1) may be an insolubilized lectin specifically bindable with terminal galactose, which is reacted with the immune complex. Then, the resulting immune complex-insolubilized lectin bound product is reacted with an enzyme-labelled anti-Clq antibody, anti-C3 antibody or protein A.

The method in accordance with another embodiment of the present invention can be conducted by:

(1) reacting a complement-binding, tumor associated antigen-antibody complex present in a body fluid as an immune complex either with an insolubilized anti-complement or with an insolubilized substance specifically bindable to BFP or the antibody against BFP constituting the complex to form an immune complex-insolubilized anti-complement bound product or an immune complex-insolubilized substance bound product,

(2) reacting the immune complex-insolubilized anti-complement bound product with a predetermined amount of labelled substance specifically bindable to BFP or the antibody against BFP constituting the bound product, or reacting the immune complex-insolubilized substance bound product with a predetermined amount of labelled anti-complement, anti-antibody or protein A,

(3) separating the resulting reaction product from the unreacted labelled substance or labelled anti-complement, anti-antibody or protein A, and

(4) determining the specific activity of the reaction product or the separated unreacted reactant. Especially, the insolubilized anti-complement used in the step (1) may be an insolubilized anti-C3 antibody or anti-Clq antibody, which is reacted with the immune complex. Then the resulting immune complex-insolubilized anti-complement bound product is reacted with an enzyme-labelled anti-BFP antibody.

The present invention will be described in greater detail with reference to the following reference examples and examples.

Reference Example 1
Preparation of C3-Sepharose®4B

A 15 g quantity (dry weight) of cyanogen bromide (CNBr)-activated Sepharose® 4B (Pharmacia Fine Chemicals Inc.) was added to 1.5 liters of $10^{-3}$N aqueous solution of HCl. The mixture was allowed to stand at room temperature for 15 minutes, and the HCl solution was removed by a glass filter. The solid portion was washed with 3 liters of 0.1 M bicarbonate buffer (pH 8.0) and then added to 200 ml of 0.1 M bicarbonate buffer (pH 8.0). Subsequently 100 mg of human C3 [Biochemical Journal (England), Vol. 193 (3), pp. 963—970 (1981)] was added to the mixture, and the resulting mixture was subjected to reaction at room temperature for 2 hours with occasional stirring. The reaction mixture was filtered with a glass filter, the solid portion was added to 200 ml of 1 M aqueous monoethanolamine solution (pH 8.0), and the mixture was allowed to stand at room temperature for 2 hours. After passing the mixture through a glass filter, the solid portion was thoroughly washed with 2 liters of 0.1 M acetic acid buffer (pH 4.0) containing 0.5 M NaCl and then with 2 liters of borate buffer (pH 8.0) containing 0.5 M NaCl. The washing procedure was repeated until the optical density at 280 nm ($OD_{280}$) of the washings decreased to not higher than 0.002, whereby human C-3Sepharose® 4B was obtained. The product was preserved in 0.05 M phosphate buffer containing 0.2% BSA (bovine serum albumin) and 0.2% $NaN_3$ at 4°C before use.

Reference Example 2
(1) Preparation of anti-C3 serum

A 10 mg quantity of human C3 (same as in Reference Example 1) was dissolved in 10 ml of physiological saline, 1 ml of the solution was admixed with 1 ml of complete Freund's adjuvant, and the mixture was subcutaneously given to a rabbit at a dose of 0.2 ml. The mixture was further administered at the same dose 2 weeks thereafter and 1 month after the second immunization. One week after the last immunization, the blood was collected to obtain anti-human C3 serum.

(2) Preparation of F(ab')₂ fraction of anti-C3 antibody [F(ab')₂-anti-C3]

A 10 ml quantity of the anti-C3 serum obtained by the procedure (1) was admixed with 20 ml of 0.9% NaCl, and 10 ml of saturated ammonium sulfate was added dropwise to the mixture. The resulting mixture was centrifuged (3000 r.p.m., 15 minutes) to obtain a solid portion, which was dialyzed with PBS (phosphate buffered saline) to prepare a γ-globulin fraction. The fraction was passed through an affinity chromatographic column with C3-Sepharose® 4B obtained in Reference Example 1, followed by washing with 0.05 M phosphate buffer (pH 7.4) containing 0.14 M NaCl and elution with 0.1 M aqueous acetic acid solution containing 0.5 M NaCl to obtain purified anti-C3 antibody. The antibody was treated with 4 mg of pepsin at 37°C for 24 hours and then subjected to Sephadex® G-150 (Farmacia) column chromatography, followed by elution with 0.1 M borate buffer (pH=8.0) containing 0.5 M NaCl to obtain F(ab')₂-anti-C3 fraction.

Reference Example 3
Preparation of enzyme-labelled antibody [alkaline phosphatase-labelled F(ab')₂-anti-BFP]

F(ab')₂-anti-BFP was prepared in the same manner as in Reference Example 2. A 10 mg portion of the product and 10 mg of alkaline phosphatase (type II, bovine intestinal alkaline phosphatase crystals, SIGMA Chemical Co.) were added to 10 ml of 0.1 M phosphate buffer (pH=7.5) containing 0.5 M NaCl, 0.5 ml of 25% glutaraldehyde solution was slowly added dropwise to the mixture, and the resulting mixture was stirred at room temperature for 2 hours. With addition of 5 g of glycine, the excess of glutaraldehyde was blocked, and the mixture was dialyzed with saline. The resulting liquid was concentrated and then subjected to gel filtration on Sepharose® 6B (Farmacia) to collect an active fraction and obtain alkaline phosphatase-labelled F(ab')₂-anti-BFP.

Reference Example 4

Preparation of insolubilized F(ab')₂-anti-C3

The F(ab')₂-anti-C3 obtained in Reference Example 2 was adjusted to 25 μg/ml with 0.1 M Tris-HCl buffer (pH=8.0). The solution was placed into the wells of a microtiter plate (made of polyvinyl chloride, U-plate, Cooke Engineering Co.) in an amount of 150 μl in each well, incubated at 37°C for 1 hour and then allowed to stand at 4°C for 20 hours. After removing the solution from the wells, the wells were washed with saline three times to obtain insolubilized F(ab')₂-anti-C3. The wells were filled with a Tris-HCl buffer for sealing and preserved at 4°C. The buffer was removed immediately before use.

Reference Example 5

Preparation of insolubilized lectin (PNA-beads)

Polystyrene beads (Precision Plastic Co., Ltd., U.S.A., 6.4 mm in diameter) were washed with 1 N aqueous NaOH solution and 1 N aqueous HCl solution alternately, three times with each, and thereafter washed with distilled water until the pH of the washings became about 7. One thousand of the beads were placed into 120 ml of 0.1 M phosphate buffer containing 100 μg/ml of PNA (peanut lectin, product of E. Y. Laboratory), then allowed to stand at 4°C for 24 hours, thereafter filtered off and washed with water. Subsequently the beads were placed into 200 ml of 0.1 M phosphate buffer containing 0.2% of gelatin, then allowed to stand at 4°C for 24 hours, thereafter filtered off and washed with water to obtain insolubilized PNA. The PNA-beads were preserved in 0.05 M phosphate buffer (0.14 M NaCl, 0.2% gelatin, 0.01% NaN₃, pH=7.4).

Reference Example 6

Preparation of samples

A 2 ml quantity of blood was collected from each of healthy persons and patients with various cancers and hepatitis, thereafter coagulated and centrifuged (2000 r.p.m., 10 minutes) to obtain the serum to be tested. Similarly blood was collected with a syringe treated with heparin (500 units) and centrifuged to obtain the plasma to be tested.

Example 1

A 0.1 ml quantity of the serum obtained in Reference Example 6 was placed into each well of the microtiter plate which is the insolubilized F(ab')₂-anti-C3 prepared in Reference Example 4, and incubated at 37°C for 2 hours. After removing the reaction mixture by suction, the well was washed with physiological saline three times. Subsequently 0.1 ml of the labelled F(ab')₂-anti-BFP prepared in Reference Example 3 was placed into the well and incubated at 37°C for 2 hours. After removing the reaction mixture by suction, the well was washed with physiological saline three times. Into the well was then placed 0.1 ml of a substrate solution (0.1 M aqueous diethanolamine solution (pH=10) containing 50 mg/100 ml of p-nitrophenylphosphoric acid), followed by incubation at 37°C for 1 hour. A 0.1 ml quantity of 1 N aqueous NaOH solution was then added to stop the reaction.

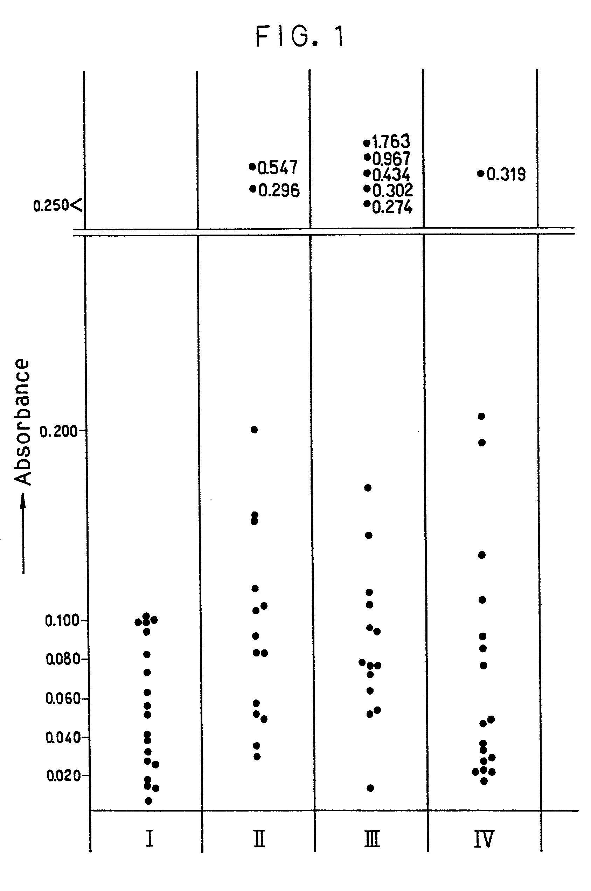

The absorbance of the reaction mixture was measured at a wavelength of 405 nm using a 1-mm-wide cell on a spectrophotometer. Fig. 1 shows the result.

With reference to Fig. 1, indicated at I is a group of 19 healthy persons (control group), at II a group of 16 patients with primary liver cancer, at III a group of 19 patients with gastric cancer, and at IV a group of 18 patients with hepatitis. The absorbances above 0.250 were plotted in the drawing along with the values.

The diagram affords diagnoses of cancers. When similar measurements were conducted for patients with hepatitis with whom free BFP is high, it was found that BFP-containing IC was low in the aggravated stage of hepatitis, whereas high BFP-containing IC measurements were obtained in the convalescent stage. The result appears to indicate that the BFP produced from regenerating liver cell is equivalent to the one derived from cancer cells, further showing that the present method is useful for clinically identifying hepatitis.

Example 2

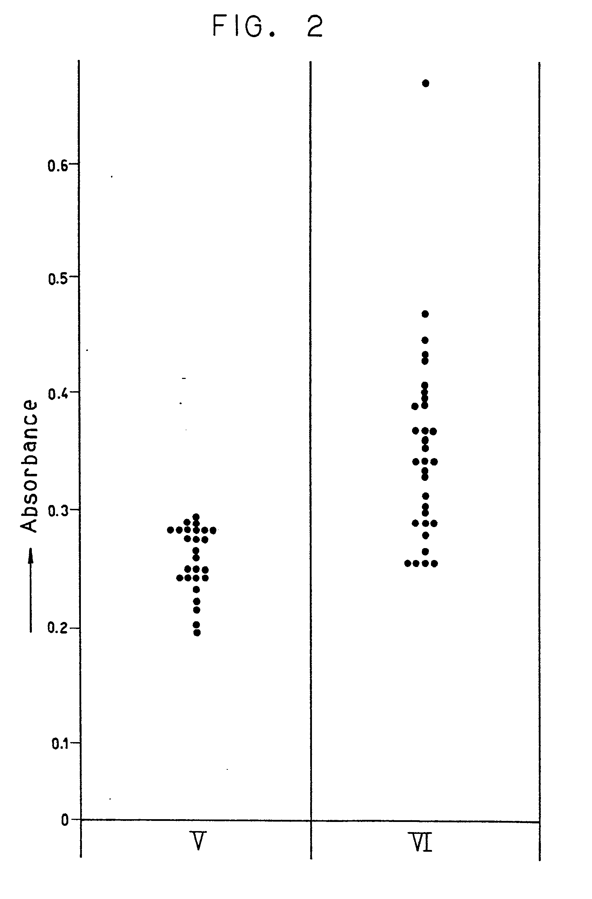

One PNA-bead obtained in Reference Example 5 and 0.5 ml of 0.05 M Tris-HCl buffer (pH=7.4) containing 0.2% gelatin, 0.15 M NaCl, 5 mM MgCl₂, 5 mM CaCl₂ and 0.01% thimerosal were added to 0.1 ml of the serum or plasma obtained in Reference Example 6, and the mixture was incubated at 25°C for 5 hours. The bead was then washed with physiological saline three times and thereafter added to a mixture of 0.1 ml of peroxidase-labelled protein A (4000-fold dilution, E. Y. Laboratory) and 0.5 ml of 0.05 M Tris-HCl buffer (pH=7.4) containing 0.2% gelatin, 0.15 M NaCl and 0.01% thimerosal, followed by incubation at 25°C for 16 hours. To the bead thereafter washed with physiological saline three times were added 0.5 ml of 0.2 M citric acid-phosphate buffer (pH=5.0) containing 0.03% H₂O₂ and 1.0 ml of physological saline containing 4 mg/ml of o-phenylenediamine, and the mixture was incubated at room temperature for 10 minutes. The reaction was terminated by addition of 2.0 ml of 1 N aqueous hydrochloric acid solution. The absorbance of the reaction mixture was measured at a wavelength of 492 nm. Fig. 2 shows the result.

With reference to Fig. 2, indicated at V is a group of 26 healthy persons (control group), and at VI a group of 32 cancer patients. The diagram provides diagnoses of cancer.

Example 3

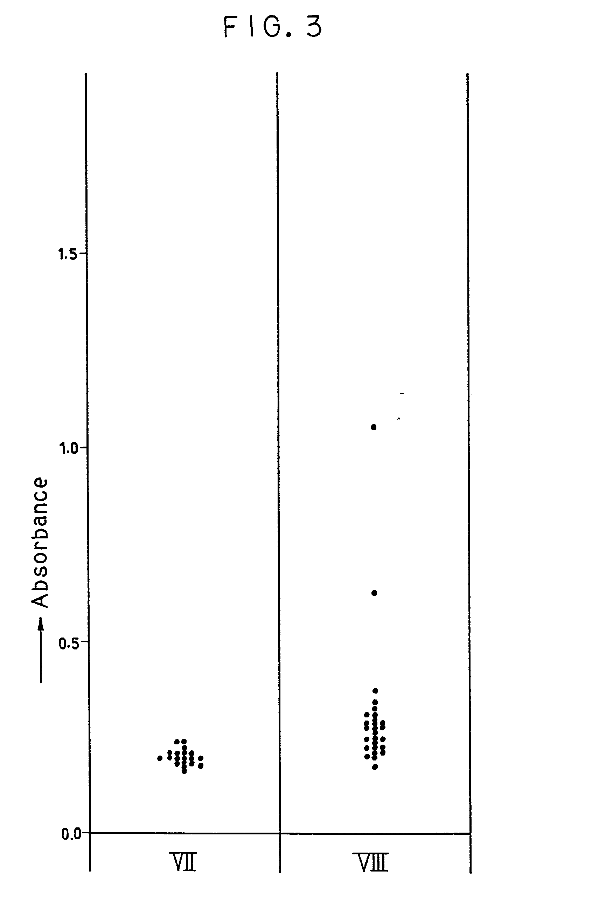

One PNA-bead obtained in Reference Example 5 and 0.5 ml of 0.05 M Tris-HCl buffer (pH=7.4) containing 0.2% gelatin, 0.15 M NaCl, 5 mM MgCl₂, 5 mM CaCl₂ and 0.01% thimerosal were added to 0.1 ml of the serum or plasma obtained in Reference Example 6, and the mixture was incubated at 25°C for 5 hours. The bead was then washed with physiological saline three times and thereafter added to a mixture of 0.1 ml of peroxidase-labelled goat anti-human C3 antibody (product of E. Y. Laboratory, X10) and 0.5 ml of 0.05 M Tris-HCl buffer (pH=7.4) containing 0.2% gelatin, 0.15 M NaCl and 0.01% thimerosal, followed by incubation at 25°C for 16 hours. To the bead thereafter washed with physiological saline three times were added 0.5 ml of 0.2 M citric acid-phosphate buffer (pH=5.0) containing 0.03% $H_2O_2$ and 1.0 ml of physiological saline containing 4 mg/ml of o-phenylenediamine, and the mixture was incubated at room temperature for 30 minutes. The reaction was terminated by addition of 2.0 ml of 1 N aqueous hydrochloric acid solution. The absorbance of the reaction mixture was measured at a wavelength of 492 nm. Fig. 3 shows the result.

With reference to Fig. 3, indicated at VII is a group of 19 healthy persons (control group), and at VIII a group of 27 cancer patients. The diagram provides diagnosis of cancer.

**Claims**

1. A method of determining an immune complex specific to a tumor associated antigen characterized in that it comprises the steps of:
    (1) reacting a complement-binding, tumor associated antigen-antibody complex present in a body fluid as an immune complex with an insolubilized anti-complement to form an immune complex-insolubilized anti-complement bound product,
    (2) reacting the immune complex-insolubilized anti-complement bound product with a predetermined amount of labelled substance specifically bindable to the tumor associated antigen selected from basic fetoprotein (BFP), α-fetoprotein (AFP), tumor associated glycolinkage (TAG) or carcinoembryonic antigen (CEA) or to the antibody thereagainst contained in the bound product,
    (3) separating the resulting reaction product from the unreacted labelled substance, and
    (4) determining the specific activity of the reaction product or the separated unreacted reactant.

2. A method as defined in claim 1 wherein the bound product of step (1) is reacted in step (2) with a predetermined amount of labelled substance specifically bindable to TAG or to the antibody against TAG contained in the bound product.

3. A method as defined in claim 1 wherein the bound product of step (1) is reacted in step (2) with a predetermined amount of labelled substance specifically bindable to BFP or to the antibody against BFP contained in the bound product.

4. A method as defined in claim 1 wherein the labelled substance used in step (2) is labelled anti-BFP, labelled anti-AFP, labelled anti-TAG, labelled anti-CEA, a labelled lectin specifically bindable with terminal galactose, a labelled lectin specifically bindable with terminal N-acetylgalactosamine or a labelled lectin specifically bindable with terminal L-fucose, these lectins being specifically bindable with TAG.

5. A method as defined in claim 2 wherein the labelled substance used in step (2) is labelled anti-TAG, a labelled lectin specifically bindable with terminal galactose, a labelled lectin specifically bindable with terminal N-acetylgalactosamine or labelled lectin specifically bindable with terminal L-fucose, these lectins being specifically bindable with TAG.

6. A method as defined in claim 1 wherein the labelled substance used in step (2) is labelled BFP, labelled AFP, labelled TAG or labelled CEA.

7. A method as defined in claim 2 wherein the labelled substance used in step (2) is labelled TAG.

8. A method as defined in claims 1 to 7 wherein the insolubilized anti-complement is an insolubilized antibody against complement component Clq or C3.

9. A method of determining an immune complex specific to a tumor associated antigen characterized in that it comprises the steps of:
    (1) reacting a complement-binding, tumor associated antigen-antibody complex present in a body fluid as an immune complex with an insolubilized substance specifically bindable to the tumor associated antigen selected from basic fetoprotein (BFP), α-fetoprotein (AFP), tumor associated glycolinkage (TAG) or carcinoembryonic antigen (CEA) or to the antibody thereagainst contained in the complex to form an immune complex-insolubilized substance bound product,
    (2) reacting the immune complex-insolubilized substance bound product with a predetermined amount of labelled anti-complement, anti-antibody or protein A,
    (3) separating the resulting reaction product from the unreacted labelled anti-complement, anti-antibody or protein A, and
    (4) determining the specific activity of the reaction product or the separated unreacted reactant.

10. A method as defined in claim 9 wherein the immune complex is reacted in step (1) with an insolubilized substance specifically bindable to TAG or to the antibody against TAG contained in the complex.

11. A method as defined in claim 9 wherein the immune complex is reacted in step (1) with an insolubilized substance specifically bindable to BFP or to the antibody against BFP contained in the complex.

12. A method as defined in claim 9 wherein the insolubilized substance used in step (1) is insolubilized anti-BFP, insolubilized anti-AFP, insolubilized anti-TAG, insolubilized anti-CEA, an insolubilized lectin specifically bindable with terminal galactose, an insolubilized lectin specifically bindable with terminal N-acetylgalactosamine or an insolubilized lectin specifically bindable with terminal L-fucose, these lectins being specifically bindable with TAG.

13. A method as defined in claim 10 wherein the insolubilized substance used in step (1) is an insolubilized anti-TAG, an insolubilized lectin specifically bindable with terminal galactose, an insolubilized lectin specifically bindable with terminal N-acetylgalactosamine or an insolubilized lectin specifically bindable with terminal L-fucose, these lectins being specifically bindable with TAG.

14. A method as defined in claim 9 wherein the insolubilized substance used in step (1) is insolubilized BFP, insolubilized AFP, insolubilized TAG or insolubilized CEA.

15. A method as defined in claim 14 wherein the insolubilized substance is an insolubilized TAG.

16. A method as defined in claims 9 to 15 wherein the labelled anti-complement is a labelled antibody against complement component Clq or C3.

17. A method as defined in claim 9 wherein the immune complex is reacted in step (1) with an insolubilized lectin specifically bindable with terminal galactose, and the resulting immune complex-insolubilized lectin bound product is reacted in step (2) with an enzyme-labelled anti-Clq antibody, anti-C3 antibody or protein A.

18. A method as defined in claim 17 wherein the immune complex is reacted in step (1) with an insolubilized peanut lectin, and the resulting immune complex-insolubilized peanut lectin bound product is reacted in step (2) with peroxydase-labelled protein A.

19. A method as defined in claim 17 wherein the immune complex is reacted in step (1) with an insolubilized peanut lectin, and the resulting immune complex-insolubilized peanut lectin bound product is reacted in step (2) with peroxydase-labelled anti-C3 antibody.

20. A method as defined in claim 1 wherein the immune complex is reacted in step (1) with an insolubilized anti-C3 antibody, and the resulting immune complex-insolubilized anti-C3 antibody bound product is reacted in step (2) with anti-BFP antibody labelled with alkaline phosphatase.

**Patentansprüche**

1. Verfahren zur Bestimmung eines für ein Tumor-assoziiertes Antigen spezifischen Immunkomplexes, gekennzeichnet durch die Stufen

(1) Umsetzung eines in einer Körperflüssigkeit als Immunkomplex anwesenden, Komplement-bindenden Komplexes aus Tumor-assoziiertem Antigen und Antikörper mit einem unlöslich gemachten anti-Komplement zur Erzeugung eines gebundenen Produktes aus Immunkomplex und unlöslich gemachtem anti-Komplement,

(2) Umsetzung des gebundenen Produktes aus Immunkomplex und unlöslich gemachtem anti-Komplement mit einer vorher bestimmten Menge eines markierten Stoffes mit spezifischer Bindungsfähigkeit an das Tumor-assoziierte Antigen, nämlich basisches Fötoprotein (BFP), α-Fötoprotein (AFP), Tumor-assoziiertes Glycolinkage (TAG) oder carcinoembryonisches Antigen (CEA), oder an den in dem gebundenen Produkt enthaltenen Antikörper dagegen,

(3) Abtrennung des erhaltenen Reaktionsproduktes vom nicht umgesetzten markierten Stoff, und

(4) Bestimmung der spezifischen Aktivität des Reaktionsproduktes oder des abgetrennten, nicht umgesetzten Reaktionsteilnehmers.

2. Verfahren nach Anspruch 1, wobei das gebundene Produkt der Stufe (1) in Stufe (2) mit einer vorbestimmten Menge eines markierten Stoffes umgesetzt wird, der spezifische Bindungsfähigkeit an TAG oder an den in dem gebundenen Produkt enthaltenen Antikörper gegen TAG aufweist.

3. Verfahren nach Anspruch 1, wobei das gebundene Produkt der Stufe (1) in Stufe (2) mit einer vorher bestimmten Menge eines markierten Stoffes umgesetzt wird, der spezifische Bindungsfähigkeit an BFP oder an den in dem gebundenen Produkt enthaltenen Antikörper gegen BFP aufweist.

4. Verfahren nach Anspruch 1, wobei der in Stufe (2) verwendete markierte Stoff markiertes anti-BFP, markiertes anti-AFP, markiertes anti-TAG, markiertes anti-CEA, ein markiertes Lectin mit spezifischer Bindungsfähigkeit an terminale Galactose, ein markiertes Lectin mit spezifischer Bindungsfähigkeit an terminales N-Acetylgalactosamin, oder ein markiertes Lectin mit spezifischer Bindungsfähigkeit an terminale L-Fucose ist, wobei diese Lectine spezifische Bindungsfähigkeit an TAG aufweisen.

5. Verfahren nach Anspruch 2, wobei der in Stufe (2) verwendete markierte Stoff markiertes anti-TAG, ein markiertes Lectin mit spezifischer Bindungsfähigkeit an terminale Galactose, ein markiertes Lectin mit spezifischer Bindungsfähigkeit an terminales N-Acetylgalactosamin oder ein markiertes Lectin mit spezifischer Bindungsfähigkeit an terminale L-Fucose ist, wobei diese Lectine spezifische Bindungsfähigkeit an TAG aufweisen.

6. Verfahren nach Anspruch 1, wobei der in Stufe (2) verwendete markierte Stoff markiertes BFP, markiertes AFP, markiertes TAG oder markiertes CEA ist.

7. Verfahren nach Anspruch 2, wobei der in Stufe (2) verwendete markierte Stoff markiertes TAG ist.

8. Verfahren nach den Ansprüchen 1 bis 7, wobei das unlöslich gemachte anti-Komplement ein unlöslich gemachter Antikörper gegen die Komplement-Komponente Clq oder C3 ist.

9. Verfahren zur Bestimmung eines für ein Tumor-assoziiertes Antigen spezifischen Immunkomplexes, gekennzeichnet durch die Stufen

(1) Umsetzung eines in einer Körperflüssigkeit als Immunkomplex anwesenden, Komplement-bindenden Komplexes aus Tumor-assoziiertem Antigen und Antikörper mit einem unlöslich gemachten Stoff mit spezifischer Bindungsfähigkeit an das Tumor-assoziierte Antigen, nämlich basisches Fötoprotein (BFP), α-Fötoprotein (AFP), Tumor-assoziiertes Glycolinkage (TAG) oder carcinoembryonisches Antigen (CEA), oder an den im Komplex enthaltenen Antikörper dagegen, zur Erzeugung eines gebundenen Produktes aus Immunkomplex und unlöslich gemachtem Stoff,

(2) Umsetzung des gebundenen Produktes aus Immunkomplex und unlöslich gemachtem Stoff mit einer vorher bestimmten Menge markiertes anti-Komplement, anti-Antikörper oder Protein A.

(3) Abtrennung des erhaltenen Reaktionsproduktes vom nicht umgesetzten markierten anti-Komplement, anti-Antikörper oder Protein A, und

(4) Bestimmung der spezifischen Aktivität des Reaktionsproduktes oder des abgetrennten nicht umgesetzten Reaktions teilnehmers.

10. Verfahren nach Anspruch 9, wobei der Immunkomplex in Stufe (1) mit einem unlöslich gemachten Stoff mit spezifischer Bindungsfähigkeit an TAG oder an den in dem Komplex enthaltenen Antikörper gegen TAG umgesetzt wird.

11. Verfahren nach Anspruch 9, wobei der Immunkomplex in Stufe (1) mit einem unlöslich gemachten Stoff mit spezifischer Bindungsfähigkeit an BFP oder an den in dem Komplex enthaltenen Antikörper gegen BFP umgesetzt wird.

12. Verfahren nach Anspruch 9, wobei der in Stufe (1) eingesetzte unlöslich gemachte Stoff unlöslich gemachtes anti-BFP, unlöslich gemachtes anti-AFP, unlöslich gemachtes anti-TAG, unlöslich gemachtes anti-CEA, ein unlöslich gemachtes Lectin mit spezifischer Bindungsfähigkeit an terminale Galactose, ein unlöslich gemachtes Lectin mit spezifischer Bindungsfähigkeit an terminales N-Acetylgalactosamin oder ein unlöslich gemachtes Lectin mit spezifischer Bindungsfähigkeit an terminale L-Fucose ist, wobei diese Lectine spezifische Bindungsfähigkeit an TAG aufweisen.

13. Verfahren nach Anspruch 10, wobei der in Stufe (1) eingesetzte unlöslich gemachte Stoff ein unlöslich gemachtes anti-TAG, ein unlöslich gemachtes Lectin mit spezifischer Bindungsfähigkeit an terminale Galactose, ein unlöslich gemachtes Lectin mit spezifischer Bindungsfähigkeit an terminales N-Acetylgalactosamin oder ein unlöslich gemachtes Lectin mit spezifischer Bindungsfähigkeit an terminale L-Fucose ist, wobei diese Lectine spezifische Bindungsfähigkeit an TAG aufweisen.

14. Verfahren nach Anspruch 9, wobei der in Stufe (1) eingesetzte unlöslich gemachte Stoff unlöslich gemachtes BFP, unlöslich gemachtes AFP, unlöslich gemachtes TAG oder unlöslich gemachtes CEA ist.

15. Verfahren nach Anspruch 14, wobei der unlöslich gemachte Stoff unlöslich gemachtes TAG ist.

16. Verfahren nach Anspruch 9 bis 15, wobei das markierte anti-Komplement ein markierter Antikörper gegen die Komplement-Komponente Clq oder C3 ist.

17. Verfahren nach Anspruch 9, wobei der Immunkomplex in Stufe (1) mit einem unlöslich gemachten Lectin mit spezifischer Bindungsfähigkeit an terminale Galactose umgesetzt wird und das erhaltene gebundene Produkt aus Immunkomplex und unlöslich gemachtem Lectin in Stufe (2) mit einem Enzym-markierten anti-Clq-Antikörper, anti-C3-Antikörper oder Protein A umgesetzt wird.

18. Verfahren nach Anspruch 17, wobei der Immunkomplex in Stufe (1) mit einem unlöslich gemachten Erdnuß-Lectin umgesetzt wird, und das erhaltene gebundene Produkt aus Immunkomplex und unlöslich gemachtem Erdnuß-Lectin in Stufe (2) mit Peroxidase-markiertem Protein A umgesetzt wird.

19. Verfahren nach Anspruch 17, wobei der Immunkomplex in Stufe (1) mit einem unlösliche gemachten Erdnuß-Lectin umgesetzt wird und das erhaltene gebunden Produkt aus Immunkomplex und unlöslich gemachtem Erdnuß-Lectin in Stufe (2) mit Peroxidase-markiertem anti-C3-Antikörper umgesetzt wird.

20. Verfahren nach Anspruch 1, wobei der Immunkomplex in Stufe (1) mit einem unlöslich gemachten anti-C3-Antikörper umgesetzt wird und das erhaltene gebundene Produkt aus Immunkomplex und unlöslich gemachtem anti-C3-Antikörper in Stufe (2) mit anti-BFP-Antikörper, markiert mit alkalischer Phosphatase, umgesetzt wird.

## Revendications

1. Procédé pour déterminer un immunocomplexe spécifique d'un antigène associé à une tumeur, caractérisé en ce qu'il comprend les étapes de:

1) réaction d'un complexe anticorps-antigène associé à une tumeur, fixant le complément, présent dans un fluide corporel en tant que complexe immun, avec un anti-complément insolubilisé pour former un produit lié comprenant l'immunocomplexe et l'anti-complément insolubilisé,

2) réaction du produit lié immunocomplexe anti-complément insolubilisé avec une quantité prédéterminée de substance marquée pouvant se lier spécifiquement à l'antigène associé à la tumeur, choisi parmi une foetoprotéine basique (BFP), une α-foetoprotéine (AFP), une glycoliaison associée à une tumeur (TAG) ou un antigène carcinoembryonique (CEA), ou à l'anticorps dirigé contre celui-là contenu dans le procédé lié,

3) séparation du produit de réaction résultant de la substance marquée qui n'a pas réagi,

4) détermination de l'activité spécifique du produit de réaction ou du réactif séparé n'ayant pas réagi.

2. Procédé selon la revendication 1, caractérisé en ce que le produit lié de l'étape 1) est traité dans l'étape 2) avec une quantité prédéterminée de substance marquée pouvant se lier de façon spécifique à une TAG ou à l'anticorps dirigé contre la TAG comprenant le produit lié.

3. Procédé selon la revendication 1, caractérisé en ce que le produit lié de l'étape 1) est traité dans l'étape 2) avec une quantité prédéterminée de substance marquée pouvant se lier spécifiquement à une BFP ou à l'anticorps dirigé contre la BFP comprenant le produit lié.

4. Procédé selon la revendication 1, caractérisé en ce que la substance marquée utilisée dans l'étape 2) est une anti-BFP marquée, une anti-AFP marquée, une anti-TAG marquée, un anti-CEA marqué, une lectine marquée pouvant se lier spécifiquement avec un galactose terminal, une lectine marquée pouvant se lier spécifiquement avec une N-acétylgalactosamine terminale ou une lectine marquée pouvant se lier spécifiquement avec un L-fucose terminal, ces lectines pouvant se lier spécifiquement avec TAG.

5. Procédé selon la revendication 2, caractérisé en ce que la substance marquée utilisée dans l'étape 2) est une anti-TAG marquée, une lectine marquée pouvant se lier spécifiquement avec un galactose terminal, une lectine marquée pouvant se lier spécifiquement avec une N-acétylgalactosamine terminale ou une lectine pouvant se lier spécifiquement avec un L-fucose terminal, ces lectines pouvant se lier spécifiquement avec TAG.

6. Procédé selon la revendication 1, caractérisé en ce que la substance marquée utilisée dans l'étape 2) est une BFP marquée, une AFP marquée, une TAG marquée ou un CEA marqué.

7. Procédé selon la revendication 2, caractérisé en ce que la substance marquée utilisée dans l'étape 2) est une TAG marquée.

8. Procédé selon les revendications 1 à 7, caractérisé en ce que l'anti-complément insolubilisé est un anticorps insolubilisé dirigé contre le composant Clq ou C3 du complément.

9. Procédé pour déterminer un immunocomplexe spécifique d'un antigène associé à une tumeur, caractérisé en ce qu'il comprend les étapes de:

1) réaction d'un complexe anticorps-antigène associé à une tumeur, fixant le complément, présent dans un fluide corporel en tant qu'immunocomplexe, avec une substance insolubilisée pouvant se lier spécifiquement à l'antigène associé à une tumeur choisi parmi une foetoprotéine basique (BFP), une α-foetoprotéine (AFP), une glycoliaison associée à une tumeur (TAG) ou un antigène carcinoembryonique (CEA), ou à l'anticorps dirigé contre celui-là contenu dans le complexe, pour former un produit lié comprenant l'immunocomplexe et la substance insolubilisée,

2) réaction du produit lié immunocomplexe-substance insolubilisée avec une quantité prédéterminée d'anti-complément marqué, d'anti-anticorps marqué ou de protéine A marquée,

3) séparation du produit de réaction résultant, de l'anticomplément, anti-anticorps ou protéine A marqué n'ayant pas réagi, et

4) détermination de l'activité spécifique du produit de réaction ou du réactif séparé n'ayant pas réagi.

10. Procédé selon la revendication 9, caractérisé en ce que l'immunocomplexe est traité dans l'étape 1) avec une substance insolubilisée pouvant se lier spécifiquement à une TAG ou à l'anticorps dirigé contre la TAG comprenant le complexe.

11. Procédé selon la revendication 9, caractérisé en ce que l'immunocomplexe est traité dans l'étape 1) avec une substance insolubilisée pouvant se lier spécifiquement à une BFP ou à l'anticorps dirigé contre la BFP comprenant le complexe.

12. Procédé selon la revendication 9, caractérisé en ce que la substance insolubilisée utilisée dans l'étape 1) est un anti-BFP insolubilisé, un anti-AFP insolubilisé, un anti-TAG insolubilisé, un anti-CEA insolubilisé, une lectine insolubilisée pouvant se lier spécifiquement avec un galactose terminal, une lectine insolubilisée pouvant se lier spécifiquement avec une N-acétyl-galactosamine terminale ou une lectine insolubilisée pouvant se lier spécifiquement avec un L-fucose terminal, ces lectines pouvant se lier spécifiquement avec une TAG.

13. Procédé selon la revendication 10, caractérisé en ce que la substance insolubilisée utilisée dans l'étape 1) est un anti-TAG insolubilisé, une lectine insolubilisée pouvant se lier spécifiquement avec un galactose terminal, une lectine insolubilisée pouvant se lier spécifiquement avec une N-acétylgalactosamine ou une lectine insolubilisée pouvant se lier spécifiquement avec un L-fucose terminal, ces lectines pouvant se lier spécifiquement avec une TAG.

14. Procédé selon la revendication 9, caractérisé en ce que la substance insolubilisée utilisée dans l'étape 1) est une BFP insolubilisée, une AFP insolubilisée, une TAG insolubilisée ou un CEA insolubilisé.

15. Procédé selon la revendication 14, caractérisé en ce que la substance insolubilisée est une TAG insolubilisée.

16. Procédé selon les revendications 9 à 15, caractérisé en ce que l'anti-complément marqué est un anticorps marqué dirigé contre un composant Clq ou C3 du complément.

17. Procédé selon la revendication 9, caractérisé en ce que l'immunocomplexe est traité dans l'étape 1) avec une lectine insolubilisée pouvant se lier spécifiquement avec un galactose terminal, et que le produit lié résultant comprenant l'immunocomplexe et la lectine insolubilisée est traité dans l'étape 2) avec un anticorps anti-Clq, un anticorps anti-C3 ou une protéine A, marqué avec une enzyme.

18. Procédé selon la revendication 17, caractérisé en ce que l'immunocomplexe est traité dans l'étape 1) avec une lectine d'arachide insolubilisée et que le produit lié résultant comprenant l'immunocomplexe et la lectine d'arachide insolubilisée est traité dans l'étape 2) avec une protéine A marquée avec une peroxydase.

19. Procédé selon la revendication 17, caractérisé en ce que l'immunocomplexe est traité dans l'étape 1) aec une lactine d'arachide insolubilisée et que le produit lié résultant comprenant l'immunocomplexe et la lectine d'arachide insolubilisée est traité dans l'étape 2) avec un anticorps anti-C3 marqué avec une peroxydase.

20. Procédé selon la revendication 1, caractérisé en ce que l'immunocomplexe est traité dans l'étape 1) avec un anticorps anti-C3 insolubilisé et que le produit lié résultant comprenant l'immunocomplexe et l'anticorps anti-C3 insolubilisé est traité dans l'étape 2) avec un anticorps anti-BFP marqué avec une phosphatase alcaline.

## FIG. 1

# FIG. 2

# FIG. 3